(19) Europäisches Patentamt
European Patent Office
Office européen des brevets

(11) EP 4 767 943 A1

(12) EUROPEAN PATENT APPLICATION

(43) Date of publication:
01.07.2026 Bulletin 2026/27

(21) Application number: 24307327.7

(22) Date of filing: 28.12.2024

(51) International Patent Classification (IPC):
A61B 5/353 (2021.01)    A61B 5/363 (2021.01)
G16H 50/20 (2018.01)

(52) Cooperative Patent Classification (CPC):
G16H 50/20; A61B 5/363; A61B 5/7264

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: Substrate HD
13006 Marseille (FR)

(72) Inventors:
• DEMARCY, Thomas
13006 MARSEILLE (FR)
• ZORODDU, Lucas
13006 MARSEILLE (FR)

(74) Representative: Plasseraud IP
104 Rue de Richelieu
CS92104
75080 Paris Cedex 02 (FR)

(54) **CARDIAC SIGNAL PROCESSING DEVICE AND METHOD**

(57)    A computer device for processing cardiac signals comprises:
- a data storage (114) arranged to receive input data sets each comprising a plurality of P wave segments each associated with an electrocardiogram track and with an acquisition time window, and a plurality of coronary sinus signals associated with the same acquisition time window and having one or more activation sequence,
- an input module comprising a plurality of classifiers, each associated with a respective atrial tachycardia class and arranged to receive an input data set, and to return a value indicating the probability that the input data set indicates an atrial tachycardia which belongs to the associated atrial tachycardia class, each classifier being further associated with a respective confusion matrix,
said atrial tachycardia classes being arranged into a hierarchy made of layers each comprising one more node, each node of a given layer being connected to at least one other node in the hierarchy, only part in the layer immediately above and/or in the layer immediately below said given layer in said hierarchy,
- a Bayesian engine comprising a prevalence matrix for pair of classifiers which atrial tachycardia classes are connected in said atrial tachycardia class hierarchy, arranged to receive a set of assessments comprising an atrial tachycardia class probability for each classifier, and to process said set of assessments according to the confusion matrices associated with the classifiers and according to said prevalence matrices in order to determine an updated set of assessments which maximizes the expression $\prod_{i=1}^{N} P(z_i|y_i) \prod_{i=1}^{N} P(y_i|yc_i)$
where $z_i$ are the updated set of assessments values, $y_i$ are the set of assessments, $yc_i$ represents the states of the child nodes of the classifier associated with $y_i$ in said atrial tachycardia class hierarchy, and N is the total number of classifiers,
said device being arranged to receive an input data set, to process it with the input module, to derive therefrom a set of assessments provided to said Bayesian engine, and to return one or more atrial tachycardia information derived from said updated set of assessments.

FIG. 1

**Description**

Technical Field

The invention relates to the field of cardiac signal processing. More specifically, it finds application in the field of atrial fibrillation treatment.

Background

In general, the treatment of atrial tachycardia (or "AT", or *"atrial tachycardia"* in English) consists of burning the area of the heart at the origin of the tachycardia. For this purpose, this area should be detected at first.

Most research articles giving an overview on the problem of location of the AT are primarily based on the analysis of the "P waves", a specific portion of the electrocardiogram (hereinafter "ECG") which corresponds to the depolarisation of the auricles.

In 1995, in the article *"Use of p wave configuration during atrial tachycardia to predict site of origin"*, Journal of the American College of Cardiology, 26(5):1315-1324, Tang et al have focused on the analysis of the polarity of the P waves in the surface electrodes to determine which auricle (the right one or the left one) is at the origin of the tachycardia. The derivations aVL and V1 have proven to be the most useful ones to differentiate the right sites from the left sites: a positive P wave in the aVL derivations predicts a right site with a 88% sensitivity and a 79% specificity. The sensitivity and the specificity of a positive P wave in the V1 derivation predicting a left site have been 93% and 88% respectively.

Since then, several studies have extended the analysis of the polarity of the P waves to predict the location of the site with a greater accuracy. In 2006, in the article *"P-Wave Morphology in Focal Atrial Tachycardia: Development of an Algorithm to Predict the Anatomic Site of Origin»*, 48: 1010-1017, Kistler et al have studied 130 focal AT to build a decision tree in order to find the origin amongst 11 possible regions, where the nodes are divided according to the polarity of the P waves in the different ECG derivations. This algorithm has succeeded in correctly classifying the origin in 93% of the 30 new ATs.

However, the criteria retained in these articles prevent the use thereof in a real-time context. Indeed, they are based on a posteriori processing allowing detecting the P waves in a very accurate manner, which is not possible in real-time. Based on the P waves determined in real-time, the results would be less conclusive and these methods unsuitable.

The Applicant has developed a solution which uses a machine-learning based locator using decision trees in EP23307441.8, which allows to determine an atrial tachycardia cardiac region of origin identifier as output.

All of these methods are known as "flat", because they aim at providing an atrial tachycardia cardiac site of origin (AT SOO) as a direct result of signal processing, without taking into account the hierarchical relationships between different types of atrial tachycardia. For example, a classifier might identify a specific type of tachycardia (e.g., peritricuspid flutter) without confirming its classification under broader categories (e.g., macro AT). This inconsistency can lead to misdiagnosis and inappropriate clinical workflow.

This can further result in a lack of clinically relevant predictions. Signals can often be noisy or ambiguous, leading to failures in low-level classifiers that attempt to detect precise AT SOOs, while in cases where the ECG leads do not return to the electrical baseline, a high-level classifier can at least indicate that the tachycardia is likely of macro origin.

The Applicant has thus searched for non-flat methods for determining AT SOO, i.e. using the hierarchical nature of AT types. This search revealed that hierarchical classification has not been specifically applied to cardiac arrhythmia diagnosis and the classification of AT SOO.

The Applicant's research led to explore the general field of hierarchical classification solutions. In doing so, the Applicant discovered an article by Silla et al. "A Survey of Hierarchical Classification Across Different Application Domains" Data Mining and Knowledge Discovery 22 (2011): 31-72 which is survey discussing hierarchical classification across various application domains, providing a comprehensive overview of existing methods and their limitations.

However, this article is a computer science and data mining oriented article, and it neither evokes AT nor the possibility, opportunity or manner to use one of the surveyed methods to determine AT SOO.

By furthering its searches, the Applicant found the article by Barutcuoglu et al. "Hierarchical Shape Classification Using Bayesian Aggregation", In IEEE International Conference on Shape Modeling and Applications 2006 (SMI'06), 44-44. IEEE, 2006. In this article, the authors highlight the issue of independent classifiers producing inconsistent predictions that violate parent-child relationships within the hierarchy.

However, this article is also a computer science and data mining oriented article, and it neither evokes AT nor the possibility, opportunity or manner to use one of the surveyed methods to determine AT SOO.

The Applicant thus concluded that, while flat methods have disadvantages, non-flat methods for determining AT SOO are simply non-existent.

The invention aims at improving the situation.

According to a first aspect, the Applicant proposes a computer device for processing cardiac signals, which comprises a data storage arranged to receive input data sets each comprising a plurality of P wave segments each associated with an electrocardiogram track and with an acquisition time window, and a plurality of coronary sinus signals associated with the same acquisition time window and having one or more activation sequence, an input module comprising a plurality of classifiers, each associated with a respective atrial tachycardia class and arranged to receive an input data set, and to return a value indicating the probability that the input data set indicates an atrial tachycardia which belongs to the associated atrial tachycardia class, each classifier being further associated with a respective confusion matrix, said atrial tachycardia classes being arranged into a hierarchy made of layers each comprising one more node, each node of a given layer being connected to at least one other node in the hierarchy, only part in the layer immediately above and/or in the layer immediately below said given layer in said hierarchy. The device further comprises a Bayesian engine comprising a prevalence matrix for pair of classifiers which atrial tachycardia classes are connected in said atrial tachycardia class hierarchy, arranged to receive a set of assessments comprising an atrial tachycardia class probability for each classifier, and to process said set of assessments according to the confusion matrices associated with the classifiers and according to said prevalence matrices in order to determine an updated set of assessments which maximizes the expression $\prod_{i=1}^{N} P(z_i|y_i) \prod_{i=1}^{N} P(y_i|yc_i)$ where $z_i$ are the updated set of assessments values, $y_i$ are the set of assessments, $yc_i$ represents the states of the child nodes of the classifier associated with $y_i$ in said atrial tachycardia class hierarchy, and N is the total number of classifiers, and said device is arranged to receive an input data set, to process it with the input module, to derive therefrom a set of assessments provided to said Bayesian engine, and to return one or more atrial tachycardia information derived from said updated set of assessments.

This device is advantageous because it provides an AT SOO determination device which uses a non-flat method. Furthermore, this device allows to operate dynamically, that is to adapt to new partial information obtained during the ablation process, such as pacing maneuvers or human interpretation.

In various embodiments, the device may present one or more of the following features:

- said device is further arranged to derive said set of assessments provided to said Bayesian engine by taking into account further input data which sets the value associated with at least one classifier in said set of assessments,
- said device is further arranged to receive a set of assessment values as an input provided to said Bayesian engine,
- said atrial tachycardia hierarchy comprises at least 3 layers and at least 6 classes, and
- at least some of said classes are associated with an atrial tachycardia site of origin.

According to a second aspect, a cardiac signals processing method is provided, which comprises:

a) receiving an input data set comprising a plurality of P wave segments each associated with an electrocardiogram track and with an acquisition time window, and a plurality of coronary sinus signals associated with the same acquisition time window and having one or more activation sequence,
b) applying one or more of a plurality of classifiers deriving a set of assessments from operation b),
d) applying a Bayesian engine to said set of assessments, said Bayesian engine comprising a prevalence matrix for pair of classifiers which atrial tachycardia classes are connected in said atrial tachycardia class hierarchy, and being arranged to process said set of assessments according to the confusion matrices associated with the classifiers and according to said prevalence matrices in order to determine an updated set of assessments which maximizes the expression $\prod_{i=1}^{N} P(z_i|y_i) \prod_{i=1}^{N} P(y_i|yc_i)$ where $z_i$ are the updated set of assessments values, $y_i$ are the set of assessments, and $yc_i$ represents the states of the child nodes of the classifier associated with $y_l$ in said atrial tachycardia class hierarchy,
e) returning one or more atrial tachycardia information derived from said updated set of assessments.

In various embodiments, this method may present one or more of the following features:

- operation c) comprises taking into account further input data which sets the value associated with at least one classifier in said set of assessments,
- operation c) comprises receiving a set of assessment values as an input provided to said Bayesian engine,
- said atrial tachycardia hierarchy comprises at least 3 layers and 6 classes, and
- at least some of said classes are associated with an atrial tachycardia site of origin.

According to a third aspect, a computer program is provided. The computer program includes instructions for adapting an apparatus to perform the method of any one of the embodiments of the second aspect.

According to a fourth aspect, a carrier is provided, which contains a computer program that includes instructions for adapting an apparatus to perform the method of any one of the embodiments of the second aspect is provided. In some embodiments, the carrier is one of an electronic signal, optical signal, radio signal, or computer readable storage medium.

According to a fifth aspect, a computer program product is provided, which comprises a non-transitory computer readable medium having stored thereon a computer program according to the third aspect.

Other features and advantages of the invention will readily appear in the following description of the drawings, which show exemplary embodiments of the invention and on which:

- Figure 1 a schematic diagram of a device according to the invention,
- Figure 2 shows an example of modules used in the computer program code of figure 1,
- Figure 3 shows an example of a function executed by the device of figure 1,
- Figure 4 shows an atrial tachycardia class hierarchy used in various embodiments, and
- Figure 5 shows a Bayesian network derived from the hierarchy of figure 4.

The drawings and the following description are comprised for the most part of positive and well-defined features. As a result, they are not only useful in understanding the invention, but they can also be used to contribute to its definition, should the need arise.

The description may make reference or use elements protected or protectable by copyright. The Applicant does not object to the reproduction of those elements in as much as it is limited to the necessary legal publications, however this should not be construed as a waiver of rights or any form of license.

In an attempt to classify the origin of the AT, the Applicant has for a long time worked based on a division of the auricles into 21 areas. More recently, in patent application EP23307441.8, the Applicant has found that it was relevant to group these areas into 4 groups for real-time application: the left auricle, the right auricle, the septum, and the lateral portion of the left auricle.

Along this research, the Applicant has devised several types of AT classifiers. One of them is the classifier of EP23307441.8, which classifies an input data set into one of 4 classes (the four regions above). As with most machine learning or deep learning techniques, this "classification" is a value which reflects the probability of belonging to each of these classes.

According to the below embodiments, the situation is a little different. Here, the focus is not so much anymore about providing a single omnipotent classifier (which corresponds to a "flat" approach as discussed in the introduction), but rather to use knowledge about the relationships between the classes in order to improve the quality of the predictions. As a result, the gist is not on the classifier type anymore, but rather on a super structure which allows to use many single class classifiers.

As this will be seen hereinbelow, the device of the invention allows determining, for a set of ECG signals and an intracardiac reference catheter commonly placed in the vein of the coronary sinus, which will hereafter be referred to by the expression "CS", information concerning the atrial tachycardia which is exhibited by signals of the input data set, including AT SOO in some cases. This determination is particularly interesting because it offers a starting point to the practitioner to search and determine the specific origin area of the AT and therefore the diseased portion of the heart. This determination is more reliable than the existing solutions because it leverages hierarchical relationships which exists between various classes of tachycardia which are associated with different AT SOOs. This is particularly advantageous, as it further allows to use different classifiers for different classes, hence both allowing simpler and more specialized types of classifiers, which in turns improves the performance of the device as a whole.

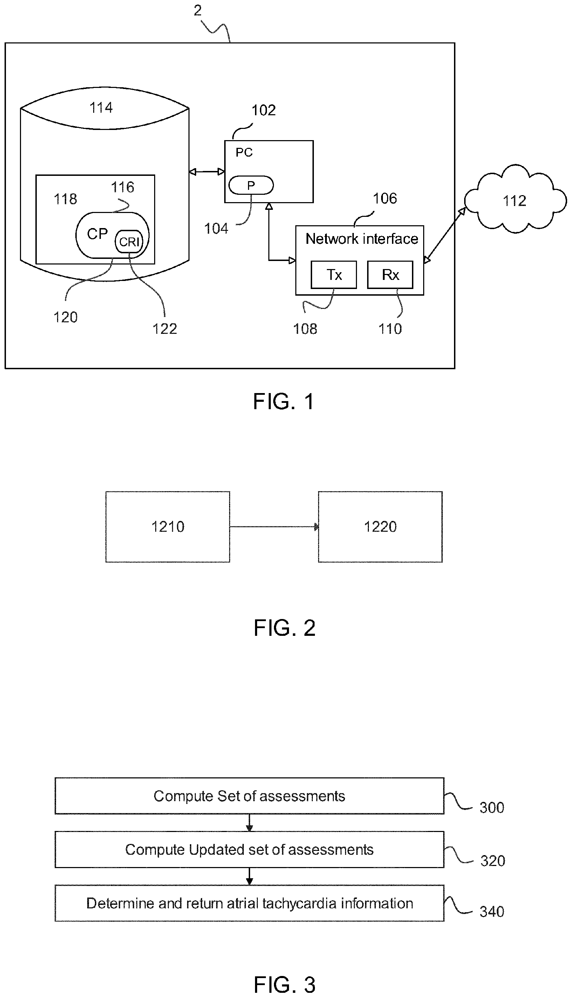

Figure 1 shows a general diagram on an embodiment of the computer device according to the invention.

The computer device shown on Figure 1 as a block diagram of an apparatus 2 (e.g., a network node, connected device, and the like), according to an embodiment. As shown in Figure 1 the apparatus may comprise: processing circuitry (PC) 102, which may include one or more processors (P) or processing circuitry 104; a network interface 106 comprising a transmitter (Tx) 108 and a receiver (Rx) 110 for enabling the apparatus to transmit data to and receive data from other computing devices connected to a network 112 (e.g., an Internet Protocol (IP) network) to which network interface 106 is connected; and a local storage unit (a.k.a., "data storage system") 114, which may include one or more non-volatile storage devices and/or one or more volatile storage devices.

In embodiments where PC 102 includes a programmable processor, a computer program product (CPP) 116 may be provided. CPP 116 includes a computer readable medium (CRM) 118 storing a computer program (CP) 120 comprising computer readable instructions (CRI) 122. CRM 118 may be a non-transitory computer readable medium, such as, magnetic media (e.g., a hard disk), optical media, memory devices (e.g., random access memory, flash memory), and the like.

The one or more processors or processing circuitry 104 include any means known for performing automated calculus, such as CPUs, GPUs, CPUs and/or GPUs grids, remote calculus grids, specifically configured FPGAs, specifically configured ASICs, specialized chips such as SOCs or NOCs, AI specialized chips, etc.

In an embodiment, data storage 114 stores input data sets as input. Each input data set comprises a plurality of P wave segments each associated with an electrocardiogram track and with an acquisition time window, and a plurality of segments deduced from coronary sinus signals associated with the same acquisition time window. In concrete terms, this means that a time window, typically 10 seconds before a current measurement time, is used to split an ECG with 9 tracks (deduced from a 12-track sensor 3 of which are ignored as they correspond to a linear combination of some of the other tracks) as wells as 5 CS signal tracks. In the particular case of the ECG, only the segments corresponding to a P wave are used. Since each heart rate is particular, this means that the input data set receives a variable number of P wave segments each being deduced from an ECG track. The present invention does not cover the particular method for obtaining the P wave segments and assumes that these form an input. In some embodiments, the apparatus 2 could be arranged to directly analyse the ECG tracks and to determine the P wave segments therein. By P wave segment, it should be understood that subsets of each ECG track are split inside the time window and define a segment of the latter.

As shown hereinbelow, all of the P wave segments of each data set are processed by an extractor module in order to deduce features therefrom which serve as an input to a locator module. Thus, for each input data set, an electrocardiogram feature vector comprising several thousand features will be generated.

Comparatively, the Applicant's previous invention, which is the object of a patent family based on French patent application FR2208851, discloses a feature extractor which provides 58 features. The features were chosen and tuned for their medical meaning and relevance with respect to atrial tachycardia. By contrast, the electrocardiogram feature vector may comprise over 10000 features, which do not appear to have a medical meaning *per se.* However, this high number of automatically generated features captures even more precisely the phenomena in the electrocardiogram and the CS.

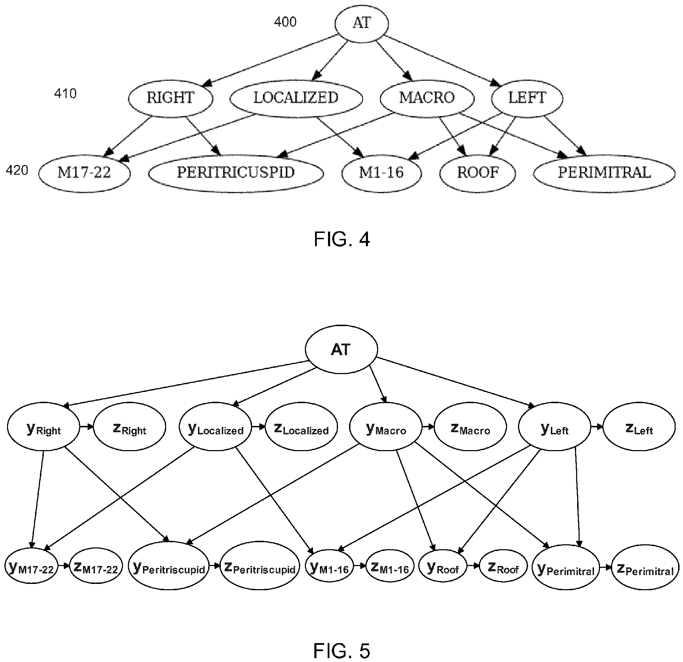

Along with the input data sets, the data storage 114 may store classifier data such as classifier confusion matrices, atrial tachycardia hierarchy information which reflect classifier links deriving from the atrial tachycardia hierarchy of figure 3 and the resulting Bayesian network of figure 4, as well as prevalence matrices which parametrize the Bayesian network in view of the trained classifiers. More details concerning the confusion matrices and the prevalence matrices will be given below.

In the example described herein, the data storage 114 may be realized in any way suitable, that is by means of a hard disk drive, a solid-state drive, a flash memory, a memory embedded in a processor, a distant storage accessible in the cloud, etc. Data storage 114 may also store any transitory data which may be generated in the course of executing the invention, as well as data resulting from the operation of the apparatus 2, possibly combined with practitioner made annotations.

Figure 4 represents an atrial tachycardia class hierarchy which is used in various embodiments. This hierarchy reflects the known medical classification of atrial tachycardias. The hierarchy of figure 3 shows 3 layers:

- a first layer 400 comprises a single class "AT",
- a second layer 410 comprises four classes "Right", "Localized", "Macro" and "Left", which are all children of the "AT" node,
- a third layer 420 comprises five classes "M17-22", "Peritricuspid", "M1-16", "Roof' and "Perimitral" .

On this figure, an arrow indicates a hierarchical relationship. For example, classes "Right", "Localized", "Macro" and "Left" are all deemed to be of the "AT" class. Similarly, class "M1-16" is deemed to be an "AT" belonging both to the "Localized" class and the "Left" class, etc.

In various embodiments, the hierarchy can have more than three layers and 6 classes or more.

Figure 5 shows the Bayesian network transposition of the hierarchy of figure 4. As can be seen on this figure, each class within a given layer is associated with a binary-valued hidden node representing actual membership to the class (referenced "y"), and a corresponding observed classifier output node (referenced "z").

Each "y to z" relationship is materialized by a confusion matrix, which provides the counts needed to estimate the probabilities used in the Bayesian network. For example, one can derive the conditional probabilities $P(z_i = 1 | y_j = 1)$ - the probability of a positive prediction given that the actual class is positive - from the confusion matrix. The confusion matrix is a 2x2 matrix in which are stored the values TN, FN, TP, FP, where TN is the number of true negatives, FN is the number of false negatives, TP is the number of true positives, and TN is the number of true negatives. These values can be obtained from predictions of a trained classifier on a validation set.

The confusion matrix allows to derive a conditional probability table, which is a 2x2 matrix of the following form:

$$\begin{bmatrix} P(z_i = 0 | y_i = 0) & P(z_i = 0 | y_i = 1) \\ P(z_i = 1 | y_i = 0) & P(z_i = 1 | y_i = 1) \end{bmatrix}, \text{ with } P(z_i = 0 | y_i = 0) = \frac{TN}{TN+FP}, \ P(z_i = 0 | y_i = 1) =$$

$$\frac{FN}{FN+TP}, P(z_i = 1 | y_i = 0) = \frac{FP}{TN+FP} \text{ and } P(z_i = 1 | y_i = 1) = \frac{TP}{FN+TP}.$$

Each "y in a layer" to "y in an upper layer" relationship is materialized by a prevalence matrix.

For each combination of parent states $y_j$, the prevalence matrix is obtained by counting the occurrences of the child states $y_i$. For example, for a binary child node $y_i$ and a binary parent node $y_j$, the counts will be: $N_{10}$, the number of

instances where $y_j = 1$ and $y_i = 0$, and $N_{11}$, the number of instances where $y_j = 1j$ and $y_i = 1$.

These counts allow to gather the necessary data to calculate the conditional probabilities for the conditional probability distribution of the child node $y_i$ based on the state of its parent node $y_j$:

- $P(y_i = 0 | y_j = 1) = 0$ - the probability of child being 1 given parent is 0, and

- $P(y_i = 1 | y_j = 1) = \frac{N_{11}}{N_{10} + N_{11}}$ - the probability of child being 1 given parent is 1

Similarly, these counts allow to calculate:

- $P(y_i = 0 | y_j = 0) = 1$ - the probability of child being 0 given parent is 0, and

- $P(y_i = 0 | y_j = 1) = \frac{N_{10}}{N_{10} + N_{11}}$ - the probability of child being 0 given parent is 1.

These formulas ensure that the parent-child hierarchy is never broken.

There will be one prevalence matrix which size will be 2x2*(number of children of the node), that is the prevalence matrix will be the concatenation of all the 2x2 prevalence matrix described above, as determined for each child given the parent.

The above elements define a local classifier per node ( or LCN) approach. It involves training a binary classifier for each node in the hierarchical structure, except for the root node. This approach distinguishes itself from the local classifier per parent node (or LCPN) approach, in which multiclass classifiers are trained to distinguish between child nodes for each parent node, as well as from the local classifier per level (or LCL) approach, where multiclass classifiers are trained for each level of the class hierarchy.

The LCN approach is combined with a Bayesian aggregation to create the Bayesian network corresponding to a class hierarchy. As will appear below, the posterior distribution is inferred using maximum a posteriori probability (MAP) estimate.

This combination of method choices presents several advantages. First, from a clinical perspective, it removes inconsistencies in classification. Furthermore, from a methodological point of view, using LCN overcomes the non-mandatory leaf node prediction (or NMLNP) problem. NMLNP is a blocking problem where, for example, a lower level is predicted only if the confidence in the prediction at the current level is greater than a threshold. Finally, as discussed above, it allows for a quick and reliable "non-flat" method using binary classifiers.

Figure 2 shows an exemplary schematic of computer program 120.

The computer program (CP) 120 comprises two main modules which work hand-in-hand:

- An input module 1210 which receives the input data sets and determines a set of assessments, and
- A Bayesian engine 1220 which receives the set of assessments and determines an updated set of assessments and returns atrial tachycardia information.

Figure 3 shows an example of a function executed by the device of figure 1.

In a first operation 300, the input module 1210 receives the input data set for which atrial tachycardia information is sought, and it computes a set of assessments. As described above, the set of assessments is obtained by applying each individual classifier, *i.e.* all the "y" classifiers of figure 5, to the input data set.

In some embodiments, the classifiers may be designed to receive different types of inputs, e.g. some classifiers may not use the coronary sinus signal. In this case, the input module 1210 reformats or reshapes the input data set so that it may be processed by each classifier.

Furthermore, the input data set may be complemented by further input data which sets the value associated with at least one classifier in the set of assessments. In this case, the concerned classifier may be skipped by the input module 1210. This can be interesting in a case where the practitioner knows a specific class to a given input data set.

For example, a practitioner may be fully convinced that a given input data set is associated with a right class tachycardia. In this case, he may indicate it and the value associated with the right class in the set of assessments will be "1". This is advantageous because, if the practitioner is certain of a medical fact, it is better to pass it on than have the classifier possibly assess it with a probability which will be less than 1. Also, it allows to gain time since this classifier can be bypassed.

Once the set of assessments has been determined, it is processed by the Bayesian engine 1220. The Applicant has analyzed that estimating the Maximum A Posteriori (MAP) is the most interesting method to compute the updated set of assessments.

MAP estimation is a method used to find the most probable value of a hidden variable (or class) given the observed data. In other words, it identifies the hidden variable that maximizes the posterior probability. In yet other words, it is a process to determine the most probable set of hidden labels given the observed set of assessments, *i.e.* the value that the y classifiers of figure 5 should have to achieve the observed value of the z classifiers of figure 5, taking into account the confusion matrices and prevalence matrices.

By applying Baye's Theorem and assuming the independence of each z classifier with respect to the y classifiers from which it does not depend in figure 5, and by rewriting the prior probability $P(y_1, y_2, \ldots, y_N) = \prod_{i=1}^{N} P(y_i | yc_i)$ where $y_i$ are the hidden variables sought *(i.e.* the y classifiers of figure 5), and $yc_i$ denotes the states of the child nodes of $y_i$, reflecting the hierarchical dependencies, one can rewrite the MAP estimation problem as:

$$MAP = \arg max_{y_1,\ldots,y_N} \left( \prod_{i=1}^{N} P(z_i | y_i) \prod_{i=1}^{N} P(y_i | yc_i) \right)$$ where N is the total number of y classifiers

This approach allows for collaborative error correction among the base classifiers while respecting the hierarchical structure of the classes, and it provides a point estimate, which can be more interpretable than a full posterior distribution.

After the updated set of assessments has been determined, the practitioner has access to the list of the y classifier values. This allows to assess which type of atrial tachycardia, if any, is exhibited by the input signal. This can be done by selecting the classes with the highest values and/or those which exceed a chosen threshold etc. They can thereafter be returned as atrial tachycardia information in an operation 340. Furthermore, if the updated assessment values allow it, the atrial tachycardia information can comprise an AT SOO.

Advantageously, operation 340 may comprise an interaction with the practitioner. For example, the practitioner may use the updated set of assessments to run complementary tests, or to modify certain values in the updated set of values and rerun the method with these values as further input, as described above, in order to drive a completed assessment. This shows a particular advantage of the invention as it allows to post process its results with ease and with limited processing costs since only the MAP has to be rerun.

While various embodiments of the present disclosure are described herein, it should be understood that they have been presented by way of example only, and not limitation. Thus, the breadth and scope of the present disclosure should not be limited by any of the above-described exemplary embodiments. Moreover, any combination of the above-described elements in all possible variations thereof is encompassed by the disclosure unless otherwise indicated herein or otherwise clearly contradicted by context.

Additionally, while the processes described above and illustrated in the drawings are shown as a sequence of steps, this was done solely for the sake of illustration. Accordingly, it is contemplated that some steps may be added, some steps may be omitted, the order of the steps may be rearranged, and some steps may be performed in parallel.

Some embodiments concern a computer-implemented method for determining atrial tachycardia cardiac site of origin (AT SOO):

Embodiment X. A computer-implemented method for determining atrial tachycardia (AT) cardiac site of origin (SOO), the method comprising:

a) receiving an input data set comprising a plurality of P wave segments, each associated with an electrocardiogram track and with an acquisition time window, and a plurality of coronary sinus signals associated with the same acquisition time window and having one or more activation sequences;

b) applying hierarchical classification to the received input data set, said classification comprising a plurality of classifiers, each associated with a respective AT class, and returning a value indicating the probability that the input data set indicates an AT which belongs to the associated AT class, each classifier being further associated with a respective confusion matrix, said AT classes arranged into a hierarchy configured as a plurality of layers, wherein each layer of the plurality of layers comprises one more nodes and each node of a given layer is connected to at least one other node in the hierarchy in a layer immediately above and/or in a layer immediately below the given layer in the hierarchy;

c) deriving from the returned value a set of assessments;

d) applying a Bayesian engine to the derived set of assessments, said Bayesian engine comprising a prevalence matrix for pairs of classifiers which AT classes are connected in said AT class hierarchy, and processing said set of assessments according to the confusion matrices associated with the classifiers and according to said prevalence matrices in order to determine an updated set of assessments; and

e) deriving from said updated set of assessments AT information useful for determining AT cardiac SOO.

Embodiment X1. The method according to Embodiment X, wherein the updated set of assessments are determined to maximize the expression $\prod_{i=1}^{N} P(z_i | y_i) \prod_{i=1}^{N} P(y_i | yc_i)$ where $z_i$ are the updated set of assessments values, $y_i$ are the set of assessments, and $yc_i$ represents the states of the child nodes of the classifier associated with $y_i$ in said

atrial tachycardia class hierarchy.

Embodiment X2. The method according to Embodiment X, wherein said AT hierarchy comprises at least 3 layers and at least 6 classes.

Embodiment X3. The method according to Embodiment X, wherein at least some of said classes are associated with an AT cardiac SOO.

Some embodiments concern a computing device:

Embodiment Y. A computing device comprising:

processing circuitry; and
a memory containing instructions executable by the processing circuitry for determining atrial tachycardia (AT) cardiac site of origin (SOO), the computing device operative to:

a) receive an input data set comprising a plurality of P wave segments, each associated with an electrocardiogram track and with an acquisition time window, and a plurality of coronary sinus signals associated with the same acquisition time window and having one or more activation sequences;
b) apply hierarchical classification to the received input data set, said classification comprising a plurality of classifiers, each associated with a respective AT class, and return a value indicating the probability that the input data set indicates an AT which belongs to the associated AT class, each classifier being further associated with a respective confusion matrix, said AT classes arranged into a hierarchy configured as a plurality of layers, wherein each layer of the plurality of layers comprises one more nodes and each node of a given layer is connected to at least one other node in the hierarchy in a layer immediately above and/or in a layer immediately below the given layer in the hierarchy;
c) derive from the returned value a set of assessments;
d) apply a Bayesian engine to the derived set of assessments, said Bayesian engine comprising a prevalence matrix for pairs of classifiers which AT classes are connected in said AT class hierarchy, and process said set of assessments according to the confusion matrices associated with the classifiers and according to said prevalence matrices in order to determine an updated set of assessments; and
e) derive from said updated set of assessments AT information useful for determining AT cardiac SOO.

Embodiment Y1. The computing device according to Embodiment Y, wherein the updated set of assessments are determined to maximize the expression $\prod_{i=1}^{N} P(z_i|y_i) \prod_{i=1}^{N} P(y_i|yc_i)$ where $z_i$ are the updated set of assessments values, $y_i$ are the set of assessments, and $yc_i$ represents the states of the child nodes of the classifier associated with $y_i$ in said atrial tachycardia class hierarchy.

## Claims

1. A computer device for processing cardiac signals, comprising:

- a data storage (114) arranged to receive input data sets each comprising a plurality of P wave segments each associated with an electrocardiogram track and with an acquisition time window, and a plurality of coronary sinus signals associated with the same acquisition time window and having one or more activation sequence,
- an input module (1210) comprising a plurality of classifiers, each associated with a respective atrial tachycardia class and arranged to receive an input data set, and to return a value indicating the probability that the input data set indicates an atrial tachycardia which belongs to the associated atrial tachycardia class, each classifier being further associated with a respective confusion matrix, said atrial tachycardia classes being arranged into a hierarchy made of layers each comprising one more node, each node of a given layer being connected to at least one other node in the hierarchy, only part in the layer immediately above and/or in the layer immediately below said given layer in said hierarchy,
- a Bayesian engine (1220) comprising a prevalence matrix for pair of classifiers which atrial tachycardia classes are connected in said atrial tachycardia class hierarchy, arranged to receive a set of assessments comprising an atrial tachycardia class probability for each classifier, and to process said set of assessments according to the confusion matrices associated with the classifiers and according to said prevalence matrices in order to determine an updated set of assessments which maximizes the expression $\prod_{i=1}^{N} P(z_i|y_i) \prod_{i=1}^{N} P(y_i|yc_i)$ where $z_i$ are the updated set of assessments values, $y_i$ are the set of assessments, $yc_i$ represents the states of the child nodes of the classifier associated with $y_i$ in said atrial tachycardia class hierarchy, and N is the total number of classifiers,

said device being arranged to receive an input data set, to process it with the input module (1210), to derive therefrom a set of assessments provided to said Bayesian engine (1220), and to return one or more atrial tachycardia information derived from said updated set of assessments.

2. Device according to claim 1, in which said device is further arranged to derive said set of assessments provided to said Bayesian engine (1220) by taking into account further input data which sets the value associated with at least one classifier in said set of assessments.

3. Device according to claim 1, in which said device is further arranged to receive a set of assessment values as an input provided to said Bayesian engine (1220).

4. Device according to one of the preceding claims, in which said atrial tachycardia hierarchy comprises at least 3 layers and at least 6 classes.

5. Device according to one of the preceding claims, in which at least some of said classes are associated with an atrial tachycardia site of origin.

6. A cardiac signals processing method, comprising:

a) receiving an input data set comprising a plurality of P wave segments each associated with an electrocardiogram track and with an acquisition time window, and a plurality of coronary sinus signals associated with the same acquisition time window and having one or more activation sequence,
b) applying one or more of a plurality of classifiers (), each associated with a respective atrial tachycardia class and arranged to receive an input data set, and to return a value indicating the probability that the input data set indicates an atrial tachycardia which belongs to the associated atrial tachycardia class, each classifier being further associated with a respective confusion matrix, said atrial tachycardia classes being arranged into a hierarchy made of layers each comprising one more node, each node of a given layer being connected to at least one other node in the hierarchy, only part in the layer immediately above and/or in the layer immediately below said given layer in said hierarchy,
c) deriving a set of assessments from operation b),
d) applying a Bayesian engine (1220) to said set of assessments, said Bayesian engine (1220) comprising a prevalence matrix for pair of classifiers which atrial tachycardia classes are connected in said atrial tachycardia class hierarchy, and being arranged to process said set of assessments according to the confusion matrices associated with the classifiers and according to said prevalence matrices in order to determine an updated set of assessments which maximizes the expression $\prod_{i=1}^{N} P(z_i|y_i) \prod_{i=1}^{N} P(y_i|yc_i)$ where $z_i$ are the updated set of assessments values, $y_i$ are the set of assessments, and $yc_i$ represents the states of the child nodes of the classifier associated with $y_l$ in said atrial tachycardia class hierarchy,
e) returning one or more atrial tachycardia information derived from said updated set of assessments.

7. Method according to claim 6, in which operation c) comprises taking into account further input data which sets the value associated with at least one classifier in said set of assessments.

8. Method according to claim 6, in which operation c) comprises receiving a set of assessment values as an input provided to said Bayesian engine (1220).

9. Method according to one of claims 6 to 8, in which said atrial tachycardia hierarchy comprises at least 3 layers and at least 6 classes.

10. Method according to one of claims 6 to 9, in which at least some of said classes are associated with an atrial tachycardia site of origin.

11. A computer program (120) comprising instructions (122) which, when executed by processing circuitry (102), causes the processing circuitry to carry out the methods of any one of claims 6 to 10.

12. A carrier containing a computer program (120) according to claim 11, in which the carrier comprises one of an electronic signals, optical signal, radio signal or computer readable storage medium.

2

**FIG. 1**

**FIG. 2**

| Compute Set of assessments | 300 |
| Compute Updated set of assessments | 320 |
| Determine and return atrial tachycardia information | 340 |

**FIG. 3**

FIG. 4

FIG. 5

| | Europäisches Patentamt European Patent Office Office européen des brevets | **EUROPEAN SEARCH REPORT** | **Application Number** EP 24 30 7327 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | TANG ET AL: "Use of P wave configuration during atrial tachycardia to predict site of origin", JOURNAL OF THE AMERICAN COLLEGE OF CARDIOLOGY, ELSEVIER, AMSTERDAM, NL, vol. 26, no. 5, 1 November 1995 (1995-11-01), pages 1315-1324, XP022150092, ISSN: 0735-1097, DOI: 10.1016/0735-1097(95)00307-X * abstract * | 1-12 | INV. A61B5/353 A61B5/363 G16H50/20 |
| A,D | CARLOS N SILLA JR ET AL: "A survey of hierarchical classification across different application domains", DATA MINING AND KNOWLEDGE DISCOVERY, KLUWER ACADEMIC PUBLISHERS, BO, vol. 22, no. 1 - 2, 7 April 2010 (2010-04-07), pages 31-72, XP019871542, ISSN: 1573-756X, DOI: 10.1007/S10618-010-0175-9 * abstract; figure 4 * | 1-12 | |
| | | | **TECHNICAL FIELDS SEARCHED (IPC)** A61B G16H |
| A,D | BARUTCUOGLU Z. ET AL: "Hierarchical Shape Classification Using Bayesian Aggregation", SHAPE MODELING AND APPLICATIONS, 2006. SMI 2006. IEEE INTERNATIONAL CO NFERENCE ON MATSUSHIMA, JAPAN 14-16 JUNE 2006, vol. 2006, 1 January 2006 (2006-01-01), pages 44-44, XP093281047, DOI: 10.1109/SMI.2006.15 ISBN: 978-0-7695-2591-4 * abstract; figure 4 * * Section 4, Results * | 1-12 | |

-/--

The present search report has been drawn up for all claims

| Place of search | Date of completion of the search | Examiner |
|---|---|---|
| Berlin | 27 May 2025 | Sleightholme, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 24 30 7327

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MURAT FATMA ET AL: "Exploring deep features and ECG attributes to detect cardiac rhythm classes", KNOWLEDGE-BASED SYSTEMS, ELSEVIER, AMSTERDAM, NL, vol. 232, 10 September 2021 (2021-09-10), XP086823263, ISSN: 0950-7051, DOI: 10.1016/J.KNOSYS.2021.107473 [retrieved on 2021-09-10] * abstract * ----- | 1-12 | |

| | | | TECHNICAL FIELDS SEARCHED (IPC) |
|---|---|---|---|

The present search report has been drawn up for all claims

| Place of search | Date of completion of the search | Examiner |
|---|---|---|
| Berlin | 27 May 2025 | Sleightholme, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 .......................................................................................

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 23307441

- FR 2208851

**Non-patent literature cited in the description**

- **TANG**. Use of p wave configuration during atrial tachycardia to predict site of origin. *Journal of the American College of Cardiology*, vol. 26 (5), 1315-1324
- **KISTLER**. *P-Wave Morphology in Focal Atrial Tachycardia: Development of an Algorithm to Predict the Anatomic Site of Origin*, vol. 48, 1010-1017

- **SILLA et al.** A Survey of Hierarchical Classification Across Different Application Domains. *Data Mining and Knowledge Discovery*, 2011, vol. 22, 31-72
- Hierarchical Shape Classification Using Bayesian Aggregation. **BARUTCUOGLU et al.** IEEE International Conference on Shape Modeling and Applications. IEEE, 2006, 44-44